# EUROPÄISCHE PATENTANMELDUNG

(11) **EP 1 101 823 A2**
(43) Veröffentlichungstag der Anmeldung: **23.05.2001**
(21) Anmeldenummer: 00122823.8
(22) Anmeldetag: 20.10.2000
(51) Int. Cl.: C12P 17/16, C12S 11/00, D06P 1/22, C12N 9/02, A61K 7/13

(54) **Verfahren zur Herstellung von Indigoderivaten sowie diese Indigoderivate enthaltende Färbemittel für Keratinfasern**

(30) Priorität: 20.11.1999 DE 19955914; 20.11.1999 DE 19955912
(71) Anmelder: Wella Aktiengesellschaft, 64274 Darmstadt (DE)
(72) Erfinder: Rozzell, David, Burbank, CA 91506 (US); Allwohn, Jürgen, 65558 Burgschwalbach (DE); Chassot, Laurent, 1724 Praroman (CH)

(57) **Zusammenfassung**

Verfahren zur Herstellung von Indigoderivaten der Formel (I), dadurch gekennzeichnet, daß in wäßriger Lösung (1) zunächst ein Tryptophanerivat der allgemeinen Formel (II) in Gegenwart von Pyridoxalphospat und eines Tryptophanase-Enzyms bei 10-50 °C enzymatisch umgesetzt wird und (2) anschließend das Reaktionsprodukt in Gegenwart von NADH und eines geeigneten Oxidase-Enzyms zu einem Indigoderivat der Formel (I) bei 10-50 °C enzymatisch oxidiert wird; 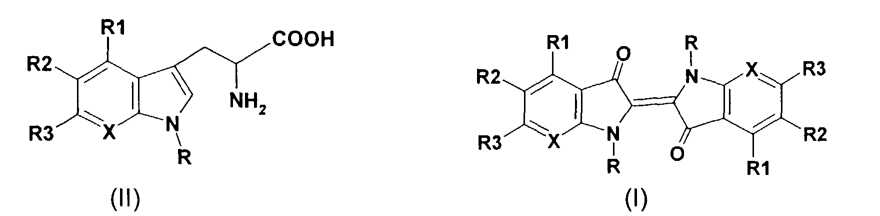 Verwendung der nach diesem Verfahren hergestellten Indigoderivate der allgemeinen Formel (II) zur Färbung keratinischer Fasern sowie Mehrkomponenten-Mittel zum Färben von keratinischen Fasern, bestehend aus einer mindestens ein Tryptophanderivat der allgemeinen Formel (II) sowie Pyridoxalphosphat enthaltenden Komponente (A), einer ein Tryptophanase-Enzym enthaltenden Komponente (B) und einer NADH sowie ein geeignetes Oxidase-Enzym enthaltenden Komponente (C), wobei das NADH und das Oxidase-Enzym auch getrennt voneinander abgepackt sein können.

## Beschreibung

Die vorliegende Erfindung betrifft ein Verfahren zur enzymatischen Herstellung von Indigoderivaten sowie diese Indigoderivate enthaltende Färbemittel für Keratinfasern, beispielsweise Wolle, Seide oder Haare und insbesondere menschliche Haare.

Die Verwendung von Indigo als blauer Farbstoff in Haarfärbemittel ist aus dem Stand der Technik seit langem bekannt. Die Synthese von Indigoderivaten (d.h. substituierten Indigoverbindungen) konnte jedoch bisher nicht in völlig befriedigender Weise gelöst werden. Es bestand daher weiterhin ein Bedarf für ein breit einsetzbares und einfach durchzuführendes Verfahren für die Herstellung von Indigo und seinen Derivaten.

Es wurde nunmehr gefunden, daß Tryptophanderivate gemäß der allgemeinen Formel (II) in Gegenwart von bestimmten Enzymen bereits bei Raumtemperatur die Herstellung einer Vielzahl von Indigoderivaten ermöglichen.

Die Synthese von unsubstituiertem Indigo mittels spezieller mikrobiologischer Verfahren ist aus der Literatur, beispielsweise der WO98/13474, der WO84/01787, der WO97/19175 und der US-PS 4520103 sowie den englischen Abstracts der JP-OS 10-257895 und JP-OS 08-089271, bekannt. Weiterhin ist es aus der WO96/08567 und der WO97/19175 bekannt, dass bei Verwendung bestimmter Enzyme ein unsubstituiertes Indigo auch aus Tryptophan hergestellt werden kann. Die enzymatische Herstellung von substituierten Indigoderivaten wird in den vorgenannten Schriften jedoch an keiner Stelle erwähnt. Ebenfalls ist aus der JP-PS 2745018 ein Verfahren zur Färbung von Haaren bekannt, bei dem die zur Färbung verwendeten Indigoverbindungen enzymatisch aus Indolen hergestellt werden. Die Möglichkeit der Verwendung von Tryptophanderivaten zur enzymatischen Herstellung von substituierten Indigoderivaten wird in der JP-PS 2745018 ebenfalls nicht beschrieben. Das in der der JP-PS 2745018 beschriebene Verfahren ist zudem nicht in jeder Hinsicht befriedigend, insbesondere in Bezug auf die Eigenschaften (Wasserunlöslichkeit und unangenehmer Geruch) und die Verfügbarkeit der verwendeten Indolderivate.

Gegenstand der vorliegenden Erfindung ist daher ein Verfahren zur Herstellung von Indigoderivaten der Formel (1), welches dadurch gekennzeichnet ist, daß in wäßriger Lösung (I) zunächst ein Tryptophanerivat der allgemeinen Formel (II) in Gegenwart von Pyridoxalphospat und eines Tryptophanase-Enzyms bei 10-50 °C enzymatisch umgesetzt wird und (2) anschließend das Reaktionsprodukt in Gegenwart von NADH und eines geeigneten Oxidase-Enzyms zu einem Indigoderivat der Formel (I) bei 10-50 °C enzymatisch oxidiert wird, 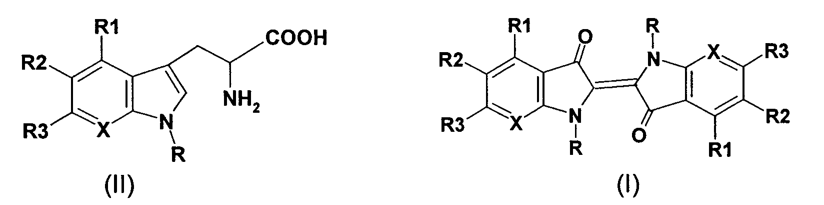 wobei in den Formeln (I) und (II) X gleich N oder einer CR4-Gruppe ist,
R gleich Wasserstoff, einer C1- bis C4-Alkylgruppe ist, und R1 bis R4 gleich oder verschieden sein können und unabhängig voneinander Wasserstoff, eine geradkettige oder verzweigte Alkylgruppe mit 1 bis 6 Kohlenstoffatomen, eine Arylgruppe, eine Difluormethylgruppe, eine Trifluormethylgruppe, ein Halogenatom (Fluor, Chlor, Brom oder Jod), eine Aminogruppe, eine C1- bis C6-Alkylaminogruppe, eine Arylaminogruppe, eine Hydroxygruppe, eine Alkoxygruppe mit 1 bis 4 Kohlenstoffatomen, eine Aryloxygruppe, eine Nitrogruppe, eine Carboxygruppe, eine Carboxyalkylgruppe, eine Carboxamidogruppe, eine Carboalkoxygruppe, eine Carboaryloxygruppe oder einen heterozyklischen Rest bedeuten, oder R1 und R2 gemeinsam beziehungsweise R2 und R3 gemeinsam eine Methylendioxygruppe bilden, unter der Bedingung, dass mindestens einer der Reste R1 bis R3 von Wasserstoff verschieden ist, wenn X gleich einer CH-Gruppe ist.

Als Tryptophanderivat der allgemeinen Formel (II) können beispielsweise genannt werden: L-5-Hydroxytryptophan, D,L-5-Hydroxytryptophan, D,L-4-Fluortryptophan, D,L-5-Fluortryptophan, D,L-6-Fluortryptophan, D,L-7-Fluortryptophan, D,L-5-Methoxytryptophan, D,L-4-Methyltryptophan, D,L-5-Methyltryptophan, D,L-6-Methyltryptophan, D,L-7-Methyltryptophan, D,L-5,6-Dihydroxytryptophan, L-5,6-Dihydroxy-tryptophan, D,L-7-Azatryptophan, 6-(Difluormethyl)-tryptophan und ß-Indazolyl-L-alanin, wobei L-5-Hydroxy-tryptophan, D,L-5-Hydroxy-tryptophan, D,L-4-Fluortryptophan, D,L-5-Fluortryptophan, D,L-6-Fluortryptophan, D,L-4-Methyltryptophan, D,L-5-Methyltryptophan, D,L-6-Methyltryptophan und D,L-7-Methyl-tryptophan, und insbesondere D,L-4-Fluortryptophan, D,L-5-Fluortryptophan, D,L-4-Methyltryptophan und D,L-7-Methyltryptophan, bevorzugt sind. Vorzugsweise werden die Tryptophanderivate der Formel (I) in ihrer L-Form eingesetzt, da das L-lsomer in Gegenwart von Tryptophanase als reaktive Komponente agiert. Besonders bevorzugt ist hierbei die Verwendung des reinen L-lsomeren, wobei unter "reinem L-Isomer" eine Komponente zu verstehen ist, welche ≤2% an D-Isomeren und mindestens 98% an L-lsomeren enthält.

Unter "Tryptophanase-Enzym" ist in der vorliegenden Anmeldung jedes Enzym oder dessen Derivat zu verstehen, das in der Lage ist, die Umsetzung von substituierten Tryptophanen zu Pyruvat, Ammoniak und dem entsprechenden Indolderivat zu katalysieren. Als Tryptophanase Enzym wird vorzugsweise ein aus *E. coli* gewonnenes Enzym verwendet; je nach eingesetztem Tryptophanderivat der Formel (II) ist es jedoch auch möglich ein aus anderen Bakterien (beispielsweise *Proteus vulgaris, Proteus rettgeri, Bacillus alvei, Enterobacter aerogenes* oder *Symbiobacterium thermophilum)* gewonnenes Tryptophanase-Enzym zu verwenden. Das Enzym wird vorzugsweise in einer Menge von 5 bis 100 units pro 1 mmol Substrat (Verbindung der Formel (II), bezogen auf den Gehalt an L-lsomer) eingesetzt. Definitionsgemäss steht 1 unit für die Menge an Enzym die zur Katalysation der Oxidation von 1µmol Tryptophanderivat pro Minute erforderlich ist.

Unter "Oxidase-Enzym" ist in der vorliegenden Anmeldung jedes Enzym oder dessen Derivat zu verstehen, das in der Lage ist, die Oxidation von Indol oder substituierten Indolen zu dem entsprechenden Indigoderivat zu katalysieren. Das Oxidase-Enzym kann sowohl in Form des vollständig oder teilweise gereinigten Proteins als auch in Form der das Enzym enthaltenden Zellen eingesetzt werden. Als Oxidase-Enzym können beispielsweise Naphthalindioxygenase, Indol-oxydase oder Toluoldioxygenase verwendet werden, wobei Naphthalin-dioxygenase bevorzugt ist. Besonders bevorzugt ist hierbei die Verwendung einer aus *Pseudomonas putida* (ATCC 17483) oder *Pseudomonas sp.* (ATCC 17484) gewonnenen Naphthalindioxygenase. Das Enzym wird vorzugsweise in einer Menge von 1 bis 200 units pro 1 mmol Substrat, vorzugsweise 10 bis 100 units pro 1 mmol Substrat, (Substrat = Verbindung der Formel (II)) eingesetzt. Definitionsgemäss steht 1 unit für die Menge an Enzym die zur Katalysation der Oxidation von 1µmol Indol pro Minute erforderlich ist.

Die Umsetzung erfolgt vorzugsweise in einem Fermenter, gegebenenfalls in Gegenwart von Glucose und/oder Aminosäuren (Stufe 1) bzw. Naphthalin (Stufe 2) und/oder unter Zufuhr von Sauerstoff oder Luftsauerstoff bei einer Temperatur von 10 bis 50 °C, wobei eine Umsetzungstemperatur von 25 bis 40 °C besonders bevorzugt.

Die Reaktionsmischung kann (sowohl in der ersten wie in der zweiten Verfahrensstufe) einen pH-Wert von etwa 2 bis 12 aufweisen, wobei ein pH-Wert von etwa 6 bis 9,5 und insbesondere 7,2 bis 9,5 bevorzugt ist.

Vorzugsweise erfolgt die Umsetzung in Gegenwart eines Kaliumsalzes und/oder eines Ammoniumsalzes, vorzugsweise Kaliumphosphat, wobei die Einsatzmenge des Kaliumsalzes und/oder Ammoniumsalzes vorzugsweise 5 bis 10 mmol pro1 mmol Substrat (Verbindung der Formel (II), bezogen auf den Gehalt an L-lsomer) beträgt.

Das in der 1. Stufe als Cofaktor wirkende Pyridoxalphosphat kann in sehr geringen Mengen, vorzugsweise in einer Menge von etwa 1 bis 50 mmol pro 1 mol Substrat (Verbindung der Formel (II), bezogen auf den Gehalt an L-lsomer), eingesetzt werden.

Das in der zweiten Verfahrensstufe als Cofaktor wirkende NADH (hydrierte Form des β-Nicotinamid-adenin-dinucleotids) kann der Reaktionsmischung in zur eingesetzen Menge an Substrat (=durch enzymatische Umsetzung des Tryptophan-derivates erhaltenes Indolderivat) stöchiometrischen Mengen, zugesetzt werden. Alternativ kann das NADH in geringeren Mengen zugesetzt werden, falls ein geeignetes Recycling-Verfahren verwendet wird. Derartige Recycling-Verfahren sind aus der Literatur bekannt, beispielsweise "Preparative Biotransformations" (S.M. Roberts, editor), 3.1.1-3.1.6, John Wiley & Sons, Chichester, U.K. (1996) sowie die dort genannte Literatur; Z. Shaked and G. M. Whitesides, J. Am. Chem. Soc. 102, 7104-5 (1980) sowie die dort genannte Literatur, und J. B. Jones and T. Takamura, Can. J. Chem. 62, 77 (1984).

Ebenfalls ist es möglich, in der 2. Stufe reduzierend wirkende Thioverbindungen, beispielsweise β-Mercaptoethanol, Natriumthioglykolat oder Dithiothreitol, zuzusetzen.

Die auf diesem Wege erhaltenen Indigoderivate der Formel (I) können als Farbstoffe in Färbemitteln, beispielsweise Haarfärbemitteln, eingesetzt werden, wobei die Indigoderivate der Formel (I) sowohl in oxidativen als auch in nicht-oxidativen Färbemitteln verwendet werden können. Die Indigoderivate der Formel (I) können entweder zunächst nach dem erfindungsgemäßen enzymatischen Verfahren hergestellt werden, um dann dem Färbemittel zugesetzt zu werden, oder aber *in situ* aus dem Tryptophanderivat der Formel (II) unmittelbar vor oder während der Färbebehandlung hergestellt werden.

Ein weiterer Gegenstand der vorliegenden Erfindung ist daher die Verwendung der nach dem erfindungsgemäßen Verfahren hergestellten Indigoderivate der Formel (I) in Färbemitteln, insbesondere in Mitteln zur Färbung keratinischer Fasern, wie zum Beispiel menschlichen Haaren, sowie ein Mehrkomponenten-Mittel (kit of parts) zur Färbung keratinischer Fasern, wie zum Beispiel Wolle, Seide oder Haaren und insbesondere menschlichen Haaren, welches dadurch gekennzeichnet ist, daß es aus einer mindestens ein Tryptophanderivat der allgemeinen Formel (II) enthaltenden Komponente (A), einer ein Tryptophanase-Enzym enthaltenden Komponente (B), wobei die Komponente (A) und/oder (B) zusätzlich Pyridoxalphosphat enthält oder das Pyridoxalphosphat separat abgepackt ist, und einer NADH sowie ein geeignetes Oxidase-Enzym enthaltenden Komponente (C), wobei das NADH und das Oxidase-Enzym auch getrennt voneinander abgepackt sein können, besteht.

Das Tryptophanderivat der allgemeinen Formel (II) ist in der Komponente (A) in einer Gesamtmenge von 0,01 bis 20 Gewichtsprozent, vorzugsweise 0,1 bis 10 Gewichtsprozent enthalten.

Das als Cofaktor wirkende Pyridoxalphosphat kann in sehr geringen Mengen, vorzugsweise in einer Menge von etwa 1 bis 50 mmol pro 1 mol Substrat (Verbindung der Formel (II), bezogen auf den Gehalt an L-lsomer), eingesetzt werden.

Das ebenfalls als Cofaktor wirkende NADH kann der Reaktionsmischung in zur eingesetzen Menge an Substrat (=durch enzymatische Umsetzung des Tryptophanderivates erhaltenes Indolderivat) stöchiometrischen Mengen, zugesetzt werden. Alternativ kann das NADH in geringeren Mengen zugesetzt werden, falls ein geeignetes Recycling-Verfahren verwendet wird.

Zur Optimierung des Farbergebnisses sowie zur Erzeugung von speziellen Farbeffekten können dem erfindungsgemäßen Färbemittel (vorzugsweise der Komponente A) weiterhin Verbindungen aus der Gruppe der direktziehenden Farbstoffe, beispielsweise aromatische Nitrofarbstoffe, Azofarbstoffe, Anthrachinon-Farbstoffe oder Triphenylmethan-Farbstoffe, alleine oder im Gemisch miteinander zugesetzt werden.
Beispiele für Nitrofarbstoffe sind Pikraminsäure, 4-(2',3'-Dihydroxypropyl)amino-3-nitro-trifluor-methylbenzol, 4,N-Ethyl-N-(2'-hydroxyethyl)-amino-1-(2'-hydroxyethyl)-amino-2-nitro-benzol, 2-Chlor-6-ethylamino-4-nitrophenol, 1-Hydroxy-2-β-hydroxyethylamino-4,6-dinitrobenzol, 4-(2'-Hydroxyethyl)-amino-3-nitro-chlorbenzol, 2-Amino-6-chlor-4-nitrophenol und 4-(2'-Hydroxyethyl)-amino-3-nitro-methylbenzol.
Beispiele für Azofarbstoffe sind 1-(2'-Methoxyphenylazo)-2-hydroxy-7-trimethyl-ammonium-naphthalin (Basic Red 76), 4-(4'-Sulfo-1-phenylazo)-1-(4"-sulfophenyl)-3-carboxy-5-hydroxypyrazolon (Acid Yellow 23), 4-Amino-4'-Bis[2"-hydroxyethyl]-amino-azobenzol (Disperse Black 9) und 1-(4'-Aminophenylazo)-2-hydroxy-7-trimethyl-ammonium-naphthalin (Basic Braun 16).
Beispiele für Anthrachinon-Farbstoffe sind 1-Methylamino-4-(2'-hydroxyethyl)amino-anthrachinon (Disperse Blue 3), 1-Amino-4-hydroxyanthrachinon (Disperse Red 15), 2-Methoxy-1,4-diamino-anthrachinon (Disperse Red 11), 1,4-Diamino-anthrachinon (Disperse Violet 1), 1-Amino-4-methylamino-anthrachinon (Disperse Violet 4), 1,4-Bis(2',3'-Dihydroxypropyl)amino-anthrachinon, 1-Methylamino-4-(amino-n-propyltrimethylammonium)-anthrachinon (Basic Blue 22), 1,4-Bis-(2-Hydroxyethyl)amino-5,8-dihydroxy-anthrachinon (Disperse Blue 7) und 1-Methylamino-4-aminopropylamino-anthrachinon (HC Blue 8).
Beispiele für Triphenylmethan-Farbstoffe sind [4-[[4-Diethylamino]phenyl][4-(ethylamino)-1-naphthalinyl]methylen]-2,5-cyclohexadien-1-yliden]-N-ethylethanamin (Basic Blue 7) und 4',4',4"-Triamino-3-methyltriphenylcarbeniumchlorid (Basic Violet 14, Fuchsin AN).

Die Menge der zugesetzten direktziehenden Farbstoffe beträgt vorzugsweise 0,01 bis 5 Gewichtsprozent, insbesondere 0,1 und 4 Gewichtsprozent.

Die Komponenten (A) bis (C) sowie das gebrauchsfertige Färbemittel können beispielsweise eine Lösung, insbesondere eine wäßrige oder wäßrig-alkoholische Lösung sein, oder in Form einer Creme, eines Gels, einer Emulsion, eines Pulvers oder Granulates vorliegen. Ihre Zusammensetzung stellt eine Mischung der Komponenten mit den für solche Zubereitungen üblichen Zusätzen dar.

Übliche kosmetische Zusätze sind zum Beispiel Lösungsmittel wie Wasser; niedere aliphatische einwertige oder mehrwertige Alkohole, deren Ester und Ether, beispielsweise Alkanole, insbesondere mit 1 bis 4 Kohlenstoffatomen in der Alkylkette, wie Ethanol, n-Propanol oder Isopropanol, Butanol, Isobutanol; zwei- oder dreiwertige Alkohole, insbesondere solche mit 2 bis 6 Kohlenstoffatomen, beispielsweise Ethylenglycol, Propylenglycol, 1,3-Propandiol, 1,4-Butandiol, 1,5-Pentandiol, 1,6-Hexandiol, 1,2,6-Hexantriol, Glycerin, Diethylenglycol, Dipropylenglycol, Polyalkylenglycole, wie Triethylenglycol, Polyethylen-glycol, Tripropylenglycol und Polypropylenglycol; niedere Alkylether von mehrwertigen Alkoholen, wie Ethylenglycol-monomethylether, Ethylen-glycol-monoethylether, Ethylenglycol-monopropylether oder Ethylenglycol-monobutylether, Diethylenglycol-monomethylether oder Diethylenglykol-monoethylether, Triethylenglycol-monomethylether oder Triethylenglycol-monoethylether; Ketone und Ketoalkohole, insbesondere solche mit 3 bis 7 Kohlenstoffatomen im Molekül, beispielsweise Aceton, Methylethylketon, Diethylketon, Methylisobutylketon, Methylphenylketon, Cyclopentanon, Cyclohexanon und Diacetonalkohol; Ether wie Dibutylether, Tetra-hydrofuran, Dioxan oder Diisopropylether; Ester wie Ethylformiat, Methylformiat, Methylacetat, Ethylacetat, Propylacetat, Butylacetat, Phenylacetat, Ethylenglycolmonoethyletheracetat oder Essigsäurehydroxyethylester; Amide wie Dimethylformamid, Dimethylacetamid oder N-Methyl-pyrrolidon; sowie Harnstoff, Tetramethylharnstoff und Thiodiglycol; weiterhin Netzmittel oder Emulgatoren aus den Klassen der anionischen, kationischen, amphoteren, nichtionogenen oder zwitterionischen ober-flächenaktiven Substanzen, wie zum Beispiel Fettalkoholsulfate, Alkyl-sulfonate, Alkylbenzolsulfonate, Alkyltrimethylammoniumsalze, Alkylbetaine, α-Olefinsulfonate, oxethylierte Fettalkohole, oxethylierte Nonylphenole, Fettsäurealkanolamine, oxethylierte Fettsäureester, Fettalkoholpolyglycolethersulfate, Alkylpolyglucoside; Verdickungsmittel wie höhere Fettalkohole, Stärke, Cellulosederivate, Vaseline, Paraffinöl, Fettsäuren und andere Fettkomponenten in emulgierter Form; wasserlösliche polymere Verdickungsmittel, beispielsweise natürliche Gummen, Guargummi, Xanthangummi, Johannisbrotkernmehl, Pektin, Dextran, Agar-Agar, Amylose, Amylopektin, Dextrine, Tone oder vollsynthetische Hydrokolloide wie Polyvinylalkohol; außerdem Pflegestoffe wie Lanolinderivate, Cholesterin, Pantothensäure, wasserlösliche kationische Polymere, Proteinderivate, Provitamine, Vitamine, Pflanzenextrakte, Zucker und Betain; Hilfsstoffe wie Elektrolyte, Antioxidantien, Fettamide, Sequestrierungsmittel, filmbildende Agentien und Konservierungsmittel.

Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Die Anwendung des erfindungsgemäßen Färbemittels kann auf verschiedene Weise erfolgen. So können die Komponenten (A), (B) und (C) (wobei das Pyridoxalphosphat der Komponente (A) und/oder (B) zugesetzt wird) vor der Anwendung miteinander vermischt werden und sofort oder aber nach einer Inkubationszeit von 15 bis 45 Minuten auf die zu färbende Faser aufgetragen werden. Man läßt das Gemisch bei etwa 15 bis 50 °C etwa 10 bis 45 Minuten lang, vorzugsweise 30 bis 40 Minuten lang, auf die Faser, beispielsweise Haare einwirken, spült sodann die Faser mit Wasser aus und trocknet sie. Im Falle einer Haarfärbung kann im Anschluß an diese Spülung das Haar mit einem Shampoo gewaschen und/oder mit einer schwachen organischen Säure, wie zum Beispiel Zitronensäure, Glycolsäure, Milchsäure, Äpfelsäure, Ascorbinsäure oder Weinsäure, nachgespült werden.

Es ist jedoch auch möglich, zunächst die Komponenten (A) und (B) (wobei das Pyridoxalphosphat der Komponente (A) und/oder (B) zugesetzt wird) gemeinsam oder einzeln nacheinander auf die Faser aufzubringen, während die Komponente (C) erst nach 10 bis 45 Minuten auf die Faser aufgetragen wird. Die Behandlung erfolgt hierbei bei einer Temperatur von 15 bis 50 °C, vorzugsweise 30 bis 45 °C. Nach einer Einwirkungszeit von weiteren 10 bis 45 Minuten, vorzugsweise 30 bis 40 Minuten, wird die Faser mit Wasser ausgespült und getrocknet. Im Falle einer Haarfärbung kann im Anschluß an diese Spülung das Haar mit einem Shampoo gewaschen und/oder mit einer schwachen organischen Säure, wie zum Beispiel Zitronensäure, Glycolsäure, Milchsäure, Äpfelsäure, Ascorbinsäure oder Weinsäure, nachgespült werden.

Die Anwendung des Färbemittels erfolgt bei einem pH-Wert von etwa 6 bis 9,5, vorzugsweise 7,2 bis 9,5, und insbesondere 8 bis 9,5.

Vorzugsweise erfolgt die Färbebehandlung in Gegenwart eines Kaliumsalzes und/oder eines Ammoniumsalzes, vorzugsweise Kaliumphosphat, wobei die Einsatzmenge des Kaliumsalzes und/oder Ammoniumsalzes vorzugsweise 50 bis 100 mmol pro10 mmol Substrat (Verbindung der Formel (II), bezogen auf den Gehalt an L-lsomer) beträgt.

Ebenfalls ist es möglich, dem Färbemittel (Komponente A) eine reduzierend wirkende Thioverbindungen, beispielsweise β-Mercaptoethanol, Natriumthioglykolat oder Dithiothreitol, zuzusetzen.

Das erfindungsgemäße Färbemittel/Färbeverfahren führt (insbesondere bei Haaren) zu Färbungen mit ausgezeichneten Echtheitseigenschaften, insbesondere was die Licht-, Wasch- und Reibechtheit anbetrifft. Hinsichtlich der färberischen Möglichkeiten bietet das erfindungsgemäße Färbemittel je nach Art und Konzentration der Verbindungen der allgemeinen Formel (II) verschiedene Farbnuancen von hellblau über türkis, blau, blauviolett bis purpur oder gold. Bemerkenswert ist hierbei die große Farbintensität und die Farbreinheit der erzielbaren Färbungen. Schließlich ist mit Hilfe des beschriebenen Haarfärbemittels auch eine Anfärbung von ergrautem und chemisch nicht vorgeschädigtem Haar problemlos und mit sehr guter Deckkraft möglich. Die hierbei erhaltenen Färbungen sind, unabhängig von der unterschiedlichen Struktur des Haares, gleichmäßig und sehr gut reproduzierbar.

Die nachfolgenden Beispiele sollen den Erfindungsgegenstand näher erläutern, ohne ihn jedoch darauf zu beschränken.

### Beispiele

| **Beispiel 1:** | Synthese von Indigoderivaten der Formel (I) |
|---|---|
| 1 mmol | Tryptophanderivat der Formel (II) gemäß Tabelle 1 |
| 0,01 mmol | Pyridoxalphosphat |
| 10 ml | Tryptophanase-Extrakt (Tryptophanase von *E. coli)* mit einem Enzymgehalt von 1unit pro Milliliter |
| 5 mmol | Kaliumphosphatpuffer |
| ad 100 ml | Wasser |

Die vorstehende Reaktionsmischung wird in einem Fermenter bei 37 °C und einem pH-Wert von 8 bis 9 in Gegenwart von Luftsauerstoff, Glucose und Aminosäuren bis zur Beendigung der Umsetzung gerührt. Anschließend wird das gebildete Indolderivat unter Zusatz von 1mmol NADH (OD₃₄₀, ca. 1,2) und 25 units Naphthalindioxygenase (von *Pseudomonas sp.* (ATCC 17484)) 24 Stunden unter Zufuhr von Luftsauerstoff und kräftigem Rühren zum Indigoderivat der Formel (I) oxidiert. Das Ergebnis der Umsetzung ist in Tabelle 1 zusammengefaßt.

**Tabelle 1**

| **Tryptophanderivat der Formel (II)** | **Farbe des erhaltenen Indigo-derivates der Formel (I)** |
|---|---|
| 4-Methyl-D,L-tryptophan | Blau |
| 5-Methyl-D,L-tryptophan | Dunkelblau |

| **Beispiel 2:** | Haarfärbemittel |
|---|---|
| Komponente (A): | |
| 1 mmol | 5,6-Dimethoxy-tryptophan |
| 0,05 mmol | Pyridoxalphosphat |
| 10 mol | Kaliumphosphatpuffer |
| ad 100 ml | Wasser |

| Komponente (B): | |
|---|---|
| 25 ml | Tryptophanase-Extrakt (Tryptophanase von *E. coli)* mit einem Enzymgehalt von 1unit pro Milliliter |
| 0,02 mmol | Pyridoxalphosphat |

| Komponente (C): | |
|---|---|
| 1 mmol | NADH (OD₃₄₀, ca. 1,2) |
| 25 units | Naphthalindioxygenase (von *Pseudomonas putida* (ATCC 17483)) |
| 10 mmol | Kaliumphosphatpuffer |
| ad 100 ml | Wasser |

Die Komponenten (A), (B) und (C) werden miteinander vermischt (pH=8-9) und auf hellblondes Haar aufgetragen. Nach einer Einwirkungszeit von 40 Minuten bei 35 bis 40 °C wird das Haar mit Wasser ausgespült und getrocknet. Das Haar hat eine violette Färbung erhalten.

| **Beispiel 3:** | Haarfärbemittel |
|---|---|
| Komponente (A): | |
| 1 mmol | 5-Methyl-tryptophan |
| 0,0025 mol | Pyridoxalphosphat |
| 10 mmol | Kaliumphosphatpuffer |
| ad 100 ml | Wasser |

| Komponente (B): | |
|---|---|
| 25 ml | Tryptophanase-Extrakt (Tryptophanase von *E. coli)* mit einem Enzymgehalt von 1unit pro Milliliter |
| 0,0025 mol | Pyridoxalphosphat |

| Komponente (C): | |
|---|---|
| 1 mmol | NADH (OD₃₄₀, ca. 1,2) |
| 25 units | Naphthalindioxygenase (von *Pseudomonas putida* (ATCC 17483)) |
| 10 mmol | Kaliumphosphatpuffer |
| ad 100 ml | Wasser |

Die Komponenten (A) und (B) werden nacheinander auf hellblondes Haar aufgetragen. Nach einer Einwirkungszeit von 30 Minuten bei 35 bis 40 °C wird Komponente (C) auf das Haar aufgetragen. Nach weiteren 30 Minuten wird das Haar mit Wasser ausgespült und getrocknet. Das Haar hat eine rote Färbung erhalten.

Die Enzymkonzentrationen werden in der vorliegenden Anmeldung in "units" ― der von der Internationalen Union für Biochemie als Standardeinheit für alle Enzyme vorgeschlagenen internationalen Einheitangegeben.

## Patentansprüche

1. Verfahren zur Herstellung von Indigoderivaten der Formel (I), dadurch gekennzeichnet, daß in wäßriger Lösung (1) zunächst ein Tryptophanerivat der allgemeinen Formel (II) in Gegenwart von Pyridoxalphospat und eines Tryptophanase-Enzyms bei 10-50 °C enzymatisch umgesetzt wird und (2) anschließend das Reaktionsprodukt in Gegenwart von NADH und eines geeigneten Oxidase-Enzyms zu einem Indigoderivat der Formel (I) bei 10-50 °C enzymatisch oxidiert wird, 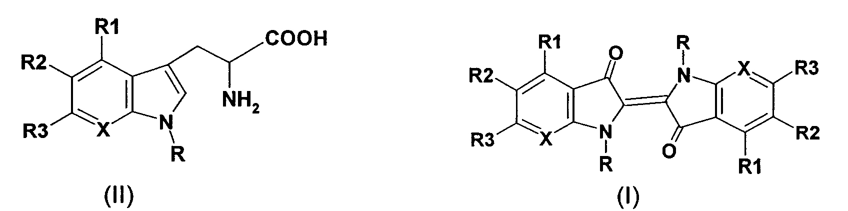 wobei in den Formeln (I) und (II) X gleich N oder einer CR4-Gruppe ist,
R gleich Wasserstoff, einer C1- bis C4-Alkylgruppe ist, und R1 bis R4 gleich oder verschieden sein können und unabhängig voneinander Wasserstoff, eine geradkettige oder verzweigte Alkylgruppe mit 1 bis 6 Kohlenstoffatomen, eine Arylgruppe, eine Difluormethylgruppe, eine Trifluormethylgruppe, ein Halogenatom (Fluor, Chlor, Brom oder Jod), eine Aminogruppe, eine C1- bis C6-Alkylaminogruppe, eine Arylaminogruppe, eine Hydroxygruppe, eine Alkoxygruppe mit 1 bis 4 Kohlenstoffatomen, eine Aryloxygruppe, eine Nitrogruppe, eine Carboxygruppe, eine Carboxyalkylgruppe, eine Carboxamidogruppe, eine Carboalkoxygruppe, eine Carboaryloxygruppe oder einen heterozyklischen Rest bedeuten, oder R1 und R2 gemeinsam beziehungsweise R2 und R3 gemeinsam eine Methylendioxygruppe bilden, unter der Bedingung, dass mindestens einer der Reste R1 bis R3 von Wasserstoff verschieden ist, wenn X gleich einer CH-Gruppe ist.

2. Verfahren nach Anspruch 1, dadurch gekennzeichnet, daß das Tryptophanderivat der Formel (II) ausgewählt ist aus L-5-Hydroxytryptophan, D,L-5-Hydroxytryptophan, D,L-4-Fluortryptophan, D,L-5-Fluortryptophan, D,L-6-Fluortryptophan, D,L-7-Fluortryptophan, D,L-5-Methoxytryptophan, D,L-4-Methyltryptophan, D,L-5-Methyltryptophan, D,L-6-Methyltryptophan, D,L-7-Methyltryptophan, D,L-5,6-Dihydroxytryptophan, L-5,6-Dihydroxy-tryptophan, D,L-7-Azatryptophan, 6-(Difluormethyl)-tryptophan und β-Indazolyl-L-alanin.

3. Verfahren nach Anspruch 1 oder 2, dadurch gekennzeichnet, daß als Oxidase-Enzym eine aus *Pseudomonas putida* (ATCC 17483) oder *Pseudomonas sp.* (ATCC 17484) gewonnene Naphthalindioxygenase verwendet wird.

4. Verfahren nach einem der Ansprüche 1 bis 3, dadurch gekennzeichnet, daß die Reaktionsmischung einen pH-Wert von 6 bis 9,5 aufweist.

5. Verfahren nach einem der Ansprüche 1 bis 4, dadurch gekennzeichnet, daß das Tryptophanase-Enzym in einer Menge von 5 bis 100 units pro 1 mmol Substrat eingesetzt wird.

6. Verfahren nach einem der Ansprüche 1 bis 5, dadurch gekennzeichnet, daß das Pyridoxalphosphat in einer Menge von 1 bis 50 mmol pro mol Substrat eingesetzt wird.

7. Verfahren nach einem der Ansprüche 1 bis 6, dadurch gekennzeichnet, daß das Oxidase-Enzym in einer Menge von 1 bis 200 units pro 1 mmol Substrat eingesetzt wird.

8. Verfahren nach einem der Ansprüche 1 bis 7, dadurch gekennzeichnet, daß das NADH in einer stöchiometrischen Menge bezogen auf die eingesetzte Menge an Substrat eingesetzt wird.

9. Verwendung der nach einem der Ansprüche 1 bis 8 hergestellten Indigoderivate zur Färbung von keratinischen Fasern.

10. Mehrkomponenten-Mittel zur Färbung von keratinischen Fasern, dadurch gekennzeichnet, daß es aus einer mindestens ein Tryptophanderivat der allgemeinen Formel (II) enthaltenden Komponente (A), einer ein Tryptophanase-Enzym sowie Pyridoxalphosphat enthaltenden Komponente (B), wobei das Tryptophanase-Enzym und das Pyridoxalphosphat auch getrennt voneinander abgepackt sein können, und einer NADH sowie ein geeignetes Oxidase-Enzym enthaltenden Komponente (C), wobei das NADH und das Oxidase-Enzym auch getrennt voneinander abgepackt sein können, besteht, 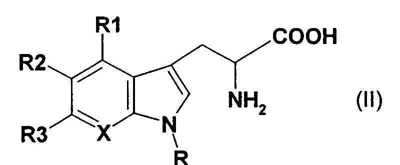 und wobei X gleich N oder einer CR4-Gruppe ist, R gleich Wasserstoff, einer C1- bis C4-Alkylgruppe ist, und R1 bis R4 gleich oder verschieden sein können und unabhängig voneinander Wasserstoff, eine geradkettige oder verzweigte Alkylgruppe mit 1 bis 6 Kohlenstoffatomen, eine Arylgruppe, eine Difluormethylgruppe, eine Trifluormethylgruppe, ein Halogenatom (Fluor, Chlor, Brom oder Jod), eine Aminogruppe, eine C1- bis C6-Alkylaminogruppe, eine Arylaminogruppe, eine Hydroxygruppe, eine Alkoxygruppe mit 1 bis 4 Kohlenstoffatomen, eine Aryloxygruppe, eine Nitrogruppe, eine Carboxygruppe, eine Carboxy-alkylgruppe, eine Carboxamidogruppe, eine Carboalkoxygruppe, eine Carboaryloxygruppe oder einen heterozyklischen Rest bedeuten, oder R1 und R2 gemeinsam beziehungsweise R2 und R3 gemeinsam eine Methylendioxygruppe bilden, unter der Bedingung, dass mindestens einer der Reste R1 bis R3 von Wasserstoff verschieden ist, wenn X gleich einer CH-Gruppe ist.

11. Mehrkomponenten-Mittel zur Färbung von keratinischen Fasern, dadurch gekennzeichnet, daß es aus einer mindestens ein Tryptophanderivat der allgemeinen Formel (II) sowie Pyridoxalphosphat enthaltenden Komponente (A), einer ein Tryptophanase-Enzym enthaltenden Komponente (B) und einer NADH sowie ein geeignetes Oxidase-Enzym enthaltenden Komponente (C), wobei das NADH und das Oxidase-Enzym auch getrennt voneinander abgepackt sein können, besteht, 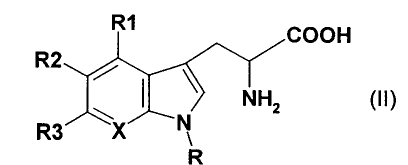 und wobei X gleich N oder einer CR4-Gruppe ist, R gleich Wasserstoff, einer C1- bis C4-Alkylgruppe ist, und R1 bis R4 gleich oder verschieden sein können und unabhängig voneinander Wasserstoff, eine geradkettige oder verzweigte Alkylgruppe mit 1 bis 6 Kohlenstoffatomen, eine Arylgruppe, eine Difluormethylgruppe, eine Trifluormethylgruppe, ein Halogenatom (Fluor, Chlor, Brom oder Jod), eine Aminogruppe, eine C1- bis C6-Alkylaminogruppe, eine Arylaminogruppe, eine Hydroxygruppe, eine Alkoxygruppe mit 1 bis 4 Kohlenstoffatomen, eine Aryloxygruppe, eine Nitrogruppe, eine Carboxygruppe, eine Carboxy-alkylgruppe, eine Carboxamidogruppe, eine Carboalkoxygruppe, eine Carboaryloxygruppe oder einen heterozyklischen Rest bedeuten, oder R1 und R2 gemeinsam beziehungsweise R2 und R3 gemeinsam eine Methylendioxygruppe bilden, unter der Bedingung, dass mindestens einer der Reste R1 bis R3 von Wasserstoff verschieden ist, wenn X gleich einer CH-Gruppe ist.

12. Mittel nach Anspruch 10 oder 11, dadurch gekennzeichnet, daß das Tryptophanderivat der Formel (II) ausgewählt ist aus L-5-Hydroxytryptophan, D,L-5-Hydroxytryptophan, D,L-4-Fluortryptophan, D,L-5-Fluortryptophan, D,L-6-Fluortryptophan, D,L-7-Fluortryptophan, D,L-5-Methoxytryptophan, D,L-4-Methyltryptophan, D,L-5-Methyltryptophan, D,L-6-Methyltryptophan, D,L-7-Methyltryptophan, D,L-5,6-Dihydroxytryptophan, L-5,6-Dihydroxy-tryptophan, D,L-7-Azatryptophan, 6-(Difluormethyl)-tryptophan und β-Indazolyl-L-alanin.

13. Mittel nach einem der Ansprüche 10 bis 12, dadurch gekennzeichnet, daß das Tryptophanderivat der Formel (II) in der Komponente (A) in einer Menge von 0,01 bis 20 Gewichtsprozent enthalten ist.

14. Mittel nach einem der Ansprüche 10 bis 13, dadurch gekennzeichnet, daß als Tryptophanase-Enzym eine aus *E. coli* gewonnene Trypthophanase verwendet wird.

15. Mittel nach einem der Ansprüche 10 bis 14, dadurch gekennzeichnet, daß als Oxidase-Enzym eine Naphthalindioxygenase, Indol-oxydase oder Toluoldioxygenase verwendet wird.

16. Mittel nach einem der Ansprüche 10 bis 15, dadurch gekennzeichnet, daß es ein Haarfärbemittel ist.
